# EUROPÄISCHE PATENTSCHRIFT

(11) **EP 1 368 004 B1**
(45) Veröffentlichungstag und Bekanntmachung des Hinweises auf die Patenterteilung: **22.04.2009**
(21) Anmeldenummer: 02719783.9
(22) Anmeldetag: 08.02.2002
(51) Int. Cl.: A61K 9/20

(54) **MUCOADHÄSIVE ZERFALLSFÄHIGE ARZNEIZUBEREITUNG ZUR WIRKSTOFFVERABREICHUNG IN DER VETERINÄR- UND HUMANMEDICIN**
MUCOADHESIVE DISPERSIBLE PHARMACEUTICAL PREPARATION FOR ACTIVE-AGENT DOSING IN VETERINARY AND HUMAN MEDICINE
PREPARATION MEDICINALE MUCOADHESIVE DEGRADABLE POUR L'ADMINISTRATION DE PRINCIPES ACTIFS EN MEDECINE HUMAINE ET VETERINAIRE

(30) Priorität: 19.02.2001 DE 10107659
(43) Veröffentlichungstag der Anmeldung: 10.12.2003
(73) Patentinhaber: LTS LOHMANN Therapie-Systeme AG, 56626 Andernach (DE)
(72) Erfinder: RADEMACHER, Tina, 53498 Bad Breisig (DE); SEIBERTS, Frank, 56548 Rheinbrohl (DE); BRANDT, Petra, 56579 Rengsdorf (DE); KRUMME, Markus, 56567 Neuwied (DE); VON FALKENHAUSEN, Christian, 53340 Meckenheim (DE)
(74) Vertreter: Flaccus, Rolf-Dieter
(86) Internationale Anmeldenummer: PCT/EP2002/001314
(87) Internationale Veröffentlichungsnummer: WO 2002/066016

(56) Entgegenhaltungen:
- WO-A-00/18365
- WO-A-00/35418
- WO-A-01/34121
- WO-A-02/02085
- DE-A- 19 646 392
- DE-A- 19 652 257
- US-A- 4 329 333

## Beschreibung

Die Erfindung betrifft mucoadhäsive, in wässrigen Medien schnell zerfallende Arzneizubereitungen zur Verabreichung von Arzneimittelwirkstoffen in schleimhautführenden Körperhöhlen des tierischen oder menschlichen Organismus.

Die orale Verabreichung von Wirkstoffen in der Veterinärmedizin ist oft mit besonderen Problemen und Mühen verbunden, da das zu behandelnde Tier häufig die Aufnahme des Medikaments verweigert. So ist beispielsweise bei Hauskatzen eine medikamentöse Therapie mittels oraler Wirkstoffapplikation äußerst aufwendig und schwierig, da sich diese der Aufnahme von Tropfen oder Tabletten stark widersetzen. Selbst wenn die zu verabreichende Arzneiform beispielsweise in Form fester Partikel unter das Tierfutter gemischt wird, führt dies meist nicht zum Erfolg, da die Tiere beim Fressen selektiv die Aufnahme der Arzneistoffpartikel vermeiden und nur das arzneistofffreie Futter aufnehmen. Falls es dennoch zu einer oralen Aufnahme kommen sollte, wird der Arzneistoff meist sofort wieder ausgespuckt, da das Tier das Medikament als Fremdkörper wahrnimmt oder dieses aufgrund seines Geschmacks für giftig oder ungenießbar hält. Daher muß der Tierhalter die Darreichungsform direkt applizieren, d. h. diese tief in den Rachen des Tieres einbringen und das Tier gegebenenfalls durch Zuhalten der Schnauze am Ausspucken hindern. Bekanntermaßen ist insbesondere der Umgang mit Katzen diesbezüglich äußerst aufwendig und erfordert in der Regel das Tragen von Schutzhandschuhen. Daher bevorzugen viele Tierhalter die Medikation durch den Tierarzt, wodurch zusätzliche Kosten entstehen. Zudem stellt die beschriebene Art der Verabreichung auch eine relativ hohle Streßbelastung für das zu behandelnde Tier dar. Ferner ist auf Grund der erwähnten Umstände nur schwer zu sicherzustellen, daß das Tier tatsächlich die vorgesehene wirkstoffdosis aufnimmt.

Aufgabe der vorliegenden Erfindung war es deshalb, Darreichungsformen bereitzustellen, durch welche die oben genannten Probleme bei der Verabreichung von Medikamenten an Tiere, insbesondere bei der oralen Verabreichung, vermieden oder zumindest vermindert werden.

Erfindungsgemäß wird diese Aufgabe gelöst durch mucoadhäsive Arzneizubereitungen nach Anspruch 1 und die in den Unteransprüchen beschriebenen bevorzugten Ausführungsformen.

Die erfindungsgemäßen Arzneizubereitungen zeichnen sich dadurch aus, daß sie eine mucoadhäsive, in wässrigen Medien zerfallsfähige Matrix aufweisen, die aus mindestens einem matrixbildenden Polymer gebildet ist und in welcher mindestens ein Wirkstoffe gelöst oder dispergiert ist; optional kann diese Matrix auch einen oder mehrere Hilfsstoffe enthalten. Ein wesentliches Merkmal der erfindungsgemäßen Arzneizubereitungen ist ferner, daß diese nach dem Einbringen in ein wässriges Medium oder in Körperflüssigkeiten schnell zerfallen, d. h. innerhalb von maximal 15 min nach dem Einbringen, oder
innerhalb von maximal 15 min nach dem Einbringen in ein wässriges Medium oder in Körperflüssigkeiten zunächst gelieren und innerhalb von maximal 30 min nach diesem Einbringen zerfallen oder erodieren.

Mucoadhäsive Darreichungsformen sind bereits mehrfach beschrieben worden, allerdings handelt es sich dabei nicht um schnell zerfallende Arzneiformen. Das US-Patent 5,750,136 beschreibt eine mucoadhäsive Darreichungsform, die an der Mucosa haftet und eine andauernde, verzögerte Wirkstoffabgabe ermöglicht. Ferner wird in US 5,908,637 eine mucoadhäsive Darreichungsform beschrieben, die zur Abgabe von Heparin geeignet ist und eine verlängerte Abgabe des Wirkstoffs an die Schleimhaut ermöglicht. Im US-Patent 5,942,243 wird eine mucoadhäsive Darreichungsform zur wirkstoffverabreichung an Tiere offenbart, welche eine anhaltende, verzögerte Wirkstoffabgabe bewirkt.
Des weiteren wird auf US 4,948,580 hingewiesen, wo eine bioadhäsive Rezeptur beschrieben wird, die als orales Freisetzungsmedium für steroide, antibakterielle und antifungizide Wirkstoffe verwendet werden kann und aus einer gefriergetrockneten Polymormischung hergestellt ist. Schließlich ist aus US 5,225,196 eine bioadhäsive Rezeptur zur kontrollierten Wirkstoffabgabe bekannt, welche ein wasserquellendes Polymer enthält, das allerdings quervernetzt und wasserunlöslich ist. Keine der genannten mucoadhäsiven Arzneiformen besitzt die Eigenschaft, in wässrigen Medien schnell (d. h. innerhalb von 15 min) zu zerfallen.

Die Druckschrift WO 00/35418 A2 hat kaubare Arzneizubereitungen zum Gegenstand, die eine bioadhäsive Komponente und eine dispergierende Brausekomponente enthalten. Bei diesen Arzneizubereitungen sorgt die aufschäumende Brausekomponente für den Zerfall der Arzneizubereitungen. Ernst durch Kauen und Speicheleinwirkung werden diese Arzneizubereitungen derart aktiviert, daß sie ein an der Mundschleimhaut haftendes Gel bilden, das den in den Zubereitungen enthaltenen Wirkstoff in der Mundhöhle zurückhalten soll.

Die Offenlegungsschrift DE 196 52 257 A1 betrifft folienförmige Zubereitungen, die einen Wirkstoff oder Aromastoff enthalten.

Die Offenlegungsschrift DE 196 46 392 A1 offenbart folienförmige Zubereitungen mit einer an einer Schleimhaut haftklebenden, einen Wirkstoff zur dosierten Abgabe enthaltenden Schicht, die vor ihrem Zerfall jedoch nicht geliert.

Die Druckschrift WO 00/18365 A2 offenbart ebenfalls folienförmige Darreichungsformen, die im Mund des Konsumenten innerhalb einer nicht näher spezifizierten Zeit zerfallen sollen.

Der wesentliche Vorteil der erfindungsgemäßen Arzneizubereitungen besteht darin, daß die mucoadhäsive, schnell zerfallende Arzneiform nur kurzzeitig mit der Zielschleimhaut des zu therapierenden Tieres in Kontakt kommen muß, um ein Festhaften der Arzneiform an der Schleimhautoberfläche bewirken. Dies hat zur Folge, daß die Arzneiform durch das Tier nicht mehr ausgespuckt oder abgeschüttelt werden kann. Erreicht wird dies im wesentlichen durch die mucoadhäsive Formulierung der Arzneizubereitung. Sobald die Arzneizubereitung Feuchtigkeit aufnimmt, wie es durch Berührung mit Schleimhäuten zu erwarten ist, setzt die klebende Wirkung der mucoadhäsiven Formulierung ein. Selbst wenn das behandelte Tier versucht, durch Kauen oder Locken die Arzneiform zu beseitigen, wird dadurch die Haftung des Systems zusätzlich unterstützt.
Auf diese Weise wird eine einfache und sichere WirkstoffApplikation ermöglicht, und es wird insbesondere die Aufnahme der vorgesehenen Wirkstoffdosen sichergestellt, da aufgrund der Schleimhaut-Haftung ein Ausstoßen oder Ausspucken der Darreichungsform nicht mehr möglich ist.

Als Zielschleimhaut kommen in erster Linie die Mundschleimhaut sowie die Schleimhäute des Rachens und der Nase in Betracht. Dies schließt nicht aus, daß die erfindungsgemäßen Arzneizubereitungen auch auf anderen Schleimhäuten des menschlichen oder tierischen Körpers, wie z. B. intestinale oder vaginale Schleimhäute, appliziert werden können. Grundsätzlich eignen sich die erfindungsgemäßen mucoadhäsiven Arzneizubereitungen zur Wirkstoffabgabe in allen schleimhautführenden Körperhöhlen, beispielsweise auch zur Anwendung im Uterus.

Nach der Applikation auf eine Schleimhautoberfläche und dem Festhaften der Arzneiform auf dieser beginnt die Arzneiform unter der Einwirkung von Feuchtigkeit oder des umgebenden wässrigen Mediums, z. B. Körperflüssigkeiten, zu zerfallen. Gleichzeitig wird dabei der in der Arzneiform enthaltende Wirkstoff freigesetzt und kann nun entweder direkt über die betreffende Schleimhaut, z. B. die Mundschleimhaut, resorbiert werden, oder durch die umgebende Körperflüssigkeiten (z. B. Speichel) weitertransportiert und an einem anderen Ort resorbiert werden. Beispielsweise können die von einer auf der Mundschleimhaut haftenden Wirkstoffzubereitung freigesetzten Wirkstoffe im Speichel gelöst oder dispergiert werden. Anschließend gelangt diese Speichel-Wirkstoff-Lösung in den Magen-Darm-Trakt, wo der/die Wirkstoff(e) resorbiert wird/werden. Vorzugsweise handelt es sich bei den erfindungsgemäßen Zubereitungen um orale Darreichungsformen.

Der Zerfallsvorgang ist innerhalb von 15 min im wesentlichen beendet, sofern die auf der Schleimhaut haftende Arzneiform während dieser Zeit von einem wässrigen Medium, z. B. einer Körperflüssigkeit, umgeben war. Gemäß bevorzugten Ausführungsformen der Erfindung sind die Arzneiformen so gestaltet, daß sie innerhalb von 3 min und besonders bevorzugt innerhalb von 60 s nach Einbringen in ein wässriges Medium zerfallen.
Die angegebenen Zerfallszeiten beruhen auf der Zerfallszeitenmessung nach Pharm. Eur. 2.9.1 "Zerfallszeiten von Tabletten und Kapseln."
Die angegebenen Zerfallszeiten können durch die Verwendung von matrixbildenden Polymeren, welche unterschiedliche Zerfalls- bzw. Löslichkeitscharakteristiken haben, in den genannten Bereichen eingestellt werden. Beispielsweise zerfällt ein "Wafer" auf der Basis von Polyvinylalkohol erheblich schneller als ein HPMC-Wafer. Durch Mischen entsprechender Polymerbestandteile läßt sich also die Zerfallszeit justieren. Darüber hinaus sind Sprengmittel bekannt, welche Wasser in die Matrix "ziehen" und diese von innen her aufsprengen. Folglich können auch solche Sprengmittel zwecks Einstellung der Zerfallszeit zugesetzt werden.

Die erfindungsgemäßen, schnell zerfallenden mucoadhäsiven Arzneizubereitungen stellen vorzugsweise flächenhafte Gebilde dar, die im folgenden als "hafer" bezeichnet werden. Weiterhin ist es auch möglich, diese Zubereitungen in Form von Pellets, Kapseln, oder Tabletten herzustellen.

Die genannten Wafer sind vergleichsweise dichte Gebilde und weisen bevorzugt eine Dichte zwischen 0,3 g/cm³ und 1,7 g/cm³ auf, besonders bevorzugt zwischen 0,5 g/cm³ und 1,5 g/cm³, und am meisten bevorzugt zwischen 0,7 g/cm³ und 1,3 g/cm³.

Die Gesamtdicke der erfindungsgemäßen Zubereitungen, insbesondere der Wafer, beträgt vorzugsweise 5 µm bis 10 mmn, bevorzugt 30 µm bis 2 mm und besonders bevorzugt 0,1 mm bis 1 mm. Die Wafer können vorteilhaft runde, ovale, ellipsenförmige, drei-, vier- oder vieleckige Formen aufweise, sie können aber auch eine beliebig gerundete Form haben.
Die Oberfläche der erfindungsgemäßen Zubereitungen ist üblicherweise glatt; jedoch kann es vorteilhaft sein, die Oberfläche mit Erhebungen und Vertiefungen zu versehen, z. B. in Gestalt von Noppen oder Rillen.

Erfindungsgemäß liegen die Zubereitungen der genannten Art in Form dünner, fester Schäume vor. Wafer in Form dünner Schäume sind vorteilhaft, da sie auf Grund ihrer großen spezifischen Oberfläche schnell anhaften, andererseits aber auch schnell zerfallen. Die Dichte dieser verfestigten Schäume liegt vorzugsweise zwischen 0,01 g/cm³ und 0,8 g/cm³, besonders bevorzugt zwischen 0,08 g/cm³ und 0,4 g/cm³, und am meisten bevorzugt zwischen 0,1 g/cm³ und 0,3 g/cm³. Bei der Berechnung der Dichte wird das durch den Gesamtkörper des Schaums ausgefüllte oder umhüllte Volumen zugrunde gelegt.
Die genannten Schäume können durch Einleiten und Dispergieren von Gasen mit Hilfe spezieller Schaumaufschlag-Vorrichtungen erzeugt werden, oder durch das Lösen von Gas unter Druck und anschließende Entspannung der Lösung.

Die mucoadhäsive, zerfallsfähige Matrix der erfindungsgemäßen Arzneizubereitungen weist mindestens ein matrixbildendes Polymer auf. Das oder die matrixbildende(n) Polymer(e) stellt/stellen einen wesentlichen Bestandteil der Matrix dar; der Polymer-Anteil beträgt mindestens 3 Gew.-% und höchstens 98 Gew.-%, vorzugsweise 7 bis 80 Gew.-%, besonders bevorzugt 20 bis 50 Gew.-%, jeweils bezogen auf die gesamte Zubereitung. Die mucoadhäsiven Eigenschaften sowie die Zerfalls-Eigenschaften werden im wesentlichen durch die Art des/der matrixbildenden Polymers/Polymere, sowie die relativen Anteile dieser Polymere in der Zubereitung bestimmt.

Als matrixbildende Polymere, welche Bestandteile einer erfindungsgemäßen mucoadhäsiven Formulierung sein können, kommen - ohne andere geeignete Rohstoffe auszuschließen - vorzugsweise folgende Polymere in Betracht:
Polyvinylalkohol (z. B. Mowiol^{®}); Cellulosederivate wie Hydroxypropylmethylcellulose, Hydroxypropylcellulose, Natrium-Carboxymethylcellulose (z. B. Walocel), Methylcellulose, Hydroxyethylcellulose und Hydroxypropylethylcellulose; Stärke und Stärkederivate; Gelatine (verschiedene Typen); Polyvinylpyrrolidon; Gummi arabicum; Pullulan; Acrylate; Polyethylenoxid, insbesondere die Typen Polyox 10, Polyox 80, Polyox 205, Polyox 301, Polyox 750 (Fa. Union Carbide); Copolymere aus Methylvinyl-Ether und Maleinsäure-Anhydrid (Gantrez-Copolymere, insbesondere die Typen ES, MS, S; Fa. ISP Global Technologies GmbH).

Neben den matrixbildenden Polymeren können der Matrix wahlweise Hilfsstoffe zugesetzt werden. Hierfür kommen Füllstoffe (z. B. SiO₂); Farbstoffe und Pigmente (z. B. Chinolingelb oder TiO₂); Sprengmittel, insbesondere Sprengmittel, die Wasser in die Matrix hineinziehen und die Matrix von innen her sprengen (z. B. Aerosil); Emulgatoren (z. B. polyethoxylierte Sorbitanfettsäureester wie TWEEN^{®} oder polyethoxylierte Fettalkohole wie BRIJ^{®}); Weichmacher (z. B. Polyethylenglykol, Glycerin); Süßstoffe (z. B. Aspartam, Saccharin); Konservierungsmittel (z. B. Sorbinsäure und deren Salze) und Aromastoffe in Betracht.

Ferner können als Hilfsstoffe auch Stabilisatoren oder Antioxidantien hinzugefügt werden, wie z. B. Ascorbylpalmitat, Natriumdisulfit, Vitamin E, Vitamin A, Vitamin C; sowohl einzeln als auch in Kombination untereinander, oder in Kombination mit anderen Hilfsstoffen.

Gemäß einer bevorzugten Ausführungsform enthalten die erfindungsgemäßen Zubereitungen mindestens einen Aromastoff und/oder mindestens einen Süßstoff und/oder mindestens einen Weichmacher.

Der Zusatz von Aromastoffen ist vorteilhaft, weil dadurch die Akzeptanz der Arzneizubereitung bei einer direkten oralen Aufnahme durch das zu behandelnde Tier verbessert wird. Ein durch den Arzneimittelwirkstoff verursachter unangenehmer Geruch oder Geschmack kann durch Beimischung eines geeigneten Geschmacks- oder Aromastoffes überlagert werden. Bei der Auswahl solcher Stoffe werden vorzugsweise die bekannten Vorlieben der zu behandelnden Tiere berücksichtig. Beispielsweise ist bekannt, daß Käse-, Sahne- und Baldrian-Aromen besonders vorteilhaft in Arzneizubereitungen eingesetzt werden können, die für die Verabreichung an Katzen bestimmt sind. Darüber hinaus können auch Fleisch-, Wurst- und Fisch-Aromen vorteilhaft eingesetzt werden, um die Bereitschaft eines Tieres zur oralen Aufnahme einer Arzneizubereitung zu erhöhen. Für bestimmte Tiergruppen sind dagegen Frucht- oder Kräuter-Aromen, wie Bananen-, Erdbeer-, Minze-, Kakao-, Nuss- oder Kaffee-Aromen; ebenso können Mischungen verschiedener Aromen eingesetzt werden.

Ebenso sollte bei der Auswahl der genannten Farbstoffe oder Pigmente auf die Wahrnehmungsfähigkeiten der zu behandelnden Tiere Rücksicht genommen werden. Beispielsweise ist eine dunkle Färbung des Systems (d. h. der Arzneizubereitung), z. B. in den Farben Blau oder Schwarz, gerade für Katzen besonders vorteilhaft, da diese nur zur Schwarz-Weiß-Unterscheidung in der Lage sind.

Die mucoadhäsive Matrix enthält ferner einen oder mehrere pharmazeutische Wirkstoffe; hierbei handelt es sich vorzugsweise um Wirkstoffe, die in der Veterinärmedizin zu therapeutischen Zwecken eingesetzt werden. Darüber hinaus kann es sich aber auch um Wirkstoffe handeln, die bei der medikamentösen Therapie in der Humanmedizin verwendet werden. Der Wirkstoff-Gehalt beträgt mindestens 0,1 Gew.-%, höchstens aber 50 Gew.-%, bezogen auf die gesamte Zubereitung.

Nachfolgend werden die im veterinärmedizinischen Bereich eingesetzten Wirkstoffe aufgeführt, welche in der erfindungsgemäßen Zubereitung enthalten sein können und mittels dieser verabreicht werden können; die Aufzählung ist nicht abschließend.
Die in der Veterinärmedizin verwendeten pharmazeutischen Wirkstoffe lassen sich in Gruppen gemäß ATCvet (Anatomical Therapeutic Chemical classification system for veterinary medicinal products); es handelt sich dabei um ein gebräuchliches Klassifizierungssystem. Mit Hilfe des ATCvet-Codes lassen sich Tierarzneimittel in therapeutische Kategorien einteilen. Damit wird das Auffinden von Tierarzaeimitteln für bestimmte Indikationen und Anwendungsweisen wesentlich erleichtert. Die Liste umfaßt folgende Gruppen und Einzelstoffe:
QA - Gastroenterologica und Betain, Pepsin, Citronensäure, Calciumcarbonat, Magnesiumsubcarbonat, Natriumchlorid, Natriumphosphat, Prifiniumbromid, Wismutsubcarbonat, Enterococcus (Streptococcus) faecium, Hefe, Methionin, Magnesiumperoxid, Pectin, Tannin, Neomycin, Biotin, Wismutsubnitrat, Papaverin, Sulfaguanidin, Calciumphosphat, Cholecalciferol, Eisengluconat, Nicotinamid, Pyridoxinhydrochlorid, Retinol, Riboflavin, Thiamin, Metronidazol, Spiramycin;
QB - Blut und Blutbildende Organe;
QC - Cardiovasculäres System; Enalapril, Furosemid, Etilefrin, Propentofyllin, Benazepril, Ramipril, Nicergolin, Pimobendan;
QD - Dermatologica; alpha-Tocopherol, Chlorophenaminmalein, Cholecalciferol, Inositol, Lecithin, Linolensäure, Mepyramein, Prednisolon, Retinol, Biotin, Griseofulvin, Cefalexin,;
QG - Gynäkologica einschließlich Geschlechtshormone; Chlorophyll, Metamizol, Nitrofurantoin, Pyridoxinhydrochlorid, Ephedrin, Chlormadinon, Metergolin, Tetrazyklin, Estriol, Megestrol, Medroxyprogesteron;
QH - Hormonales System (ohne Geschlechtshormone); Methionin, Nicotinamid, Pyridoxinhydrochlorid, Flumetason, Prednisolon, Hydroxyzin;
QJ - Antiinfectiva; Ampicillin, Amoxicillin, Clavulansäure, Cefadroxil, Cefalexin, Clindamycin, Difloxacin, Doxycyclin, Enrofloxacin, Lincomycin, Marbofloxacin, Sulfadimidin, Sulfadimethoxin, Trimethoprim, Metronidazol, Spiramycin;
QM - Muskel- und Skelettsystem; Phenylbutazon, Meloxicam, Cyanocobalamin, Dexamethason, Carprofen, Flunixin, Phenylbutazon, Pyridoxinhydrochlorid, Prednisolon, Suxibuzon, Aluminiunmagnesiumsilikat, Nifluminsäure, Thiamine;
QN - Nervensystem; Acepromazin, Clomipramin, Physostigmin, Ketamin, Selegilin, Acetylsalicylsäure, Paracetamol, Phenylbutazon, Detomedine); Anästhetika oder Sedativa wie z. B. Medetomidine, Detomidine, Dexmedetomidine; Antisedativa wie z. B. Atipamezole; Analgetika;
QP - Antiparasitika; Epsiprantel, Nitroscanat, Piperazin, Pyrantel, Oxantel, Fenbendazol, Praziquantel, Nitenpyram, Praziquantel, Febantel, Flubendazol, Milbemycinoxim, Mebendazol, Lufenuron, Carnidazol, Niclosamid, Tetramisol;
QR - Respirationssystem; Chlorophenaminmalein, Dextromethorphan, Ephedrin, Guaifenesin, Theophyllin;
QS - Sinnesorgane; Betamethason, Neomycin, Dexamethason, Gentamycin;
QV - Varia; Mitotan.

Als Wirkstoffe kommen ferner pharmakologisch aktive Substanzen in Betracht, die in den nachfolgend genannten Klassen oder Gruppen enthalten sind:
α-adrenerge Agonisten; β-adrenerge Agonisten; α-adrenerge Blocker; β-adrenerge Blocker; Alkohol-Entwöhnungsmittel; Aldose-Reductase-Inhibitoren; Anabolica; narkotische Analgetica, vorzugsweise Codeine, Morphinderivate; nicht-narkotische Analgetica, vorzugsweise Salicylate und deren Derivate; Androgene; Anaesthetica; Appetitzügler; Anthelmintica (wirksam gegen Cestoden, Nematoden, Onchocerca, Schistosomen oder Trematoden); Anti-Akne-Wirkstoffe; Anti-Allergica, Antiamöbica (amöbizide Wirkstoffe); Anti-Androgene; Wirkstoffe gegen Angina pectoris; Antiarrhythmica; anti-arteriosklerotische Wirkstoffe; anti-arthritische antirheumatische Wirkstoffe; antibakterielle Wirkstoffe (Antibiotica), vorzugsweise Aminoglycoside, Amphenicole, Ansamycine, β-Lactame (insbesondere Carbapeneme, Cephalosporine, Cephamycine, Monobactame, Oxacepheme, Penicilline), Lincosamide, Macrolide, Polypeptide, Tetracycline; synthetische antibakterielle Wirkstoffe, vorzugsweise 2,4-Diaminopyrimidine, Nitrofurane, Chinolone und -analoge, Sulfonamide, Sulfone; Anticholinergica; Anticonvulsiva; Antidepressiva, vorzugsweise bizyklische Antidepressiva, Hydrazide, Hydrazine, Pyrrolidone, tetrazyklische Antidepressiva; trizyklische Antidepressiva, polycyclische Imide; antidiabetische Mittel, vorzugsweise Biguanide, Sulfonylharnstoff-Derivate; antidiarrhöische Wirkstoffe; Antidiuretica; Anti-Estrogene; Antimykotica / fungizide Wirkstoffe, vorzugsweise Polyene; synthetische Antimykotica / fungizide Wirkstoffe, vorzugsweise Allylamine, Imidazole, Triazole; Antiglaukom-Wirkstoffe; Antigonadotropine; Wirkstoffe gegen Gicht; Antihistaminica, vorzugsweise Alkylamin-Derivate, Aminoalkyl-Ether, Ethylendiamin-Derivate, Piperazine, trizyklische Verbindungen (insbesondere Phenothiazine); antihyperlipoproteinämische Wirkstoffe (Lipidsenker), vorzugsweise Aryloxyalkansäure-Derivate (insbesondere Clofibrinsäurederivate und -analoge), Gallensäuren-sequestrierende (maskierende) Substanzen, HMG-CoA-Reductase-Inhibitoren, Nicotinsäure-Derivate, Schilddrüsenhormone und Analoge davon; antihypertonische / blutdrucksenkende Wirkstoffe, vorzugsweise Benzothiadiazin-Derivate, N-Carboxyalkyl-(Peptid/Lactam)-Derivate, Guanidin-Derivate, Hydrazine / Phthalazine, Imidazol-Derivate, quaternäre Ammoniumverbindungen, Chinazolin-Derivate, Reserpin-Derivate, Sulfonamid-Derivate; Wirkstoffe gegen Schilddrüsenüberfunktion; Wirkstoffe gegen Hypotonie; Wirkstoffe gegen Schilddrüsen-Unterfunktion; nicht-steroidale entzündungshemmende Wirkstoffe (Antiphlogistica), vorzugsweise Aminoarylcarbonsäure-Derivate, Arylessigsäure-Derivate, Arylbuttersäure-Derivate, Arylcarbonsäure-Derivate, Arylpropionsäure-Derivate, Pyrazole, Pyrazolone, Salicylsäure-Derivate, Thiazincarboxamide; Anti-Malaria-Wirkstoffe, vorzugsweise Chinin und dessen Salze, Säuren und Derivate; Anti-Migräne-Wirkstoffe; Wirkstoffe gegen Übelkeit; antineoplastische Wirkstoffe, vorzugsweise alkylierende Agenzien (insbesondere Alkylsulfonate, Aziridine, Ethylenimine und Methylmelamine, Stickstoffsenfgase, Nitrosoharnstoffe), antibiotische Wirkstoffe, Antimetabolite (insbesondere Folsäure-Analoge, Purin-Analoge, Pyrimidin-Analoge), Enzyme, Interferone, Interleukine; hormonale antineoplastische Wirkstoffe, vorzugsweise Androgene, anti-adrenale Wirkstoffe, Anti-Androgene, Anti-Estrogene (insbesondere Aromatase-Inhibitoren); antineoplastische Diätzusätze; Anti-Parkinson-Wirkstoffe; Wirkstoffe gegen Phäochromocytome; Wirkstoffe gegen Pneumocystis; Wirkstoffe zur Behandlung von Prostata-Hypertrophie; Anti-Protozoen-Wirkstoffe, vorzugsweise gegen Leishmania, Trichomonas, Trypanosoma; antipruritische Wirkstoffe; Antipsoriasis-Wirkstoffe; antipsychotische Wirkstoffe, vorzugsweise Butyrophenone, Phenothiazine, Thioxanthene, andere tricyclische Wirkstoffe, 4-Arylpiperazine, 4-Arylpiperidine; antipyretische Wirkstoffe; Mittel gegen Rickettsien; Mittel gegen Seborrhöe; Antiseptica, vorzugsweise Guanidine, Halogene und Halogen-Verbindungen, Nitrofurane, Phenole, Chinoline; antispastische / krampflösende Wirkstoffe; Antithrombotica; Antitussiva; Anti-Ulcus-Wirkstoffe; Uricostatica (Axitiurolithica); Antivenenum; antivirale Wirkstoffe, vorzugsweise Purine, Pyrimidinone; Anxiolytica, vorzugsweise Arylpiperazine, Benzodiazepin-Derivate, Carbamate; Benzodiazepin-Antagonisten; Bronchodilatatoren, vorzugsweise Ephedrin-Derivate, quaternäre Amanonium-Verbindungen, Xanthin-Derivate; Calciumkanal-Blocker, vorzugsweise Arylalkylamine, Dihydropyridin-Derivate, Piperazin-Derivate; Calcium-Regulatoren; Cardiotonica; Chelat- bzw. Komplexbildner; Cholecystokinin-Antagonisten; cholelitholytische Wirkstoffe; Choleretica; Cholinergica; Cholinesterase-Inhibitoren; Cholinesterase-Reaktivatoren; ZNS-Stimulantien; Dekongestionsmittel; prophylactische Mittel gegen Zahnkaries; Depigmentierungs-Mittel; Diuretica, vorzugsweise organische Quecksilberverbindungen, Pteridine, Purine, Steroide, Sulfonamid-Derivate, Uracile; Dopaminrezeptor-Agonisten; Wirkstoffe gegen Ectoparasiten; Enzyme, vorzugweise Verdauungsenzyme, Penicillin-inaktivierende Enzme, proteolytische Enzyme; Enzym-induzierende Wirkstoffe; steroidale und nicht-steroidale Estrogene; Magensekretion-Inhibitoren; Glucocorticoide; Gonaden-stimulierende Wirkstoffe; gonadotrope Hormone; Wachstumshormon-Inhibitoren; Wachstumshormon-Releasing-Faktor; Wachstums-Stimulantien; hämolytische Wirkstoffe; Heparin-Antagonisten; hepatoprotektive Mittel, Wirkstoffe zur Behandlung von Leberkrankheiten; Immunomodulatoren; Imounsupprimierende Wirkstoffe; IonenaustauscherHarze; Laktation-stimulierende Hormone; LH-RH-Agonisten; lipotrope Wirkstoffe; Mittel gegen Lupus erythematosus; Mineralocorticoide; Miotica; Monoaminoxidase-Inhibitoren; Mucolytica; Muskelrelaxantien; Narcotica-Antagonisten; neuroprotektive Wirkstoffe; Nootropica; Ophthalmica; Ovarialhormone; Oxytozica; Pepsin-Inhibitoren; Peristaltik-Stimulantien; Progestogene; Prolactin-Inhibitoren; Prostaglandine und -analoge; Protease-Inhibitoren; Atmungs-Stimulantien; Sklerosierungsmittel; Sedativa / Hypnotica, vorzugsweise acyclische Ureide, Alkohole, Amide, Barbitursäure-Derivate, Benzodiazepin-Derivate, Bromide, Carbamate, Chloral-Derivate, Piperidindione, Chinazolon-Derivate; Thrombolytica; thyreotrope Hormone; Uricosurica; Vasodilatatoren (cerebral); Vasodilatatoren (coronar); Vasodilatatoren (peripher); vasoprotektive Mittel; Vitamine, Vitamin-Vorstufen, Vitamin-Extrakte, Vitamin-Derivate; Wundmittel.

Die vorstehende Liste ist nicht abschließend. Sie umfaßt sowohl Wirkstoffe, die in der humanmedizinischen Therapie oder Prophylaxe angewandt werden, als auch solche, die im veterinärmedizinischen Bereich eingesetzt werden können.

Um die Wirkstoffaufnahme zu unterstützen, ist gemäß einer weiteren bevorzugten Ausführungsform der Zusatz von Agenzien vorgesehen, welche die Wirkstoffaufnahme beschleunigen (Enhancer). Als Enhancer kommen insbesondere in Betracht: Propandiol, Dexpanthenol, Ölsäure; der/die Enhancer können beispielsweise aus folgender Gruppe ausgewählt sein: Gesättigte oder ungesättigte Fettsäuren, Kohlenwasserstoffe, geradkettige oder verzweigtkettige Fettalkohole, Dimethylsulfoxid, Propylenglykol, Decanol, Dodecanol, 2-Octyldodecanol, Glycerin, Isopropylidenglycerol, Transcutol (= Diethylenglycol-monoethylether), DEET (= N,N-Diethyl-m-Toluolamid), Solketal, Ethanol oder andere Alkohole, Menthol und andere ätherische Öle oder Bestandteile ätherischer Öle, Laurinsäurediethanolamid, D-alpha-Tocopherol und Dexpanthenol; die vorstehende Aufzählung ist nicht abschließend.
Auch Kombinationen von zwei oder mehreren Enhancersubstanzen lassen sich vorteilhaft einsetzen.

Die Wirkstoffaufnahme läßt sich ferner mittels durchblutungsfördernder Stoffe verbessern, welche den erfindungsgemäßen Zubereitungen zugesetzt werden können. Hierzu zählen insbesondere Menthol, Eukalyptol, Ginkgo-Extrakt, Geranium-Öl, Campher, Krauseminzöl, Wacholderbeerenöl und Rosmarin. Diese durchblutungsfördernenden Stoffe können einzeln oder in Kombination, oder auch in Kombination mit einem oder mehreren der vorgenannten Enhancer-Stoffe eingesetzt werden.

Die erfindungsgemäßen mucoadhäsiven Zubereitungen können vorteilhaft zur Verabreichung von Wirkstoffen an Haus- oder Nutztiere oder andere Tiere, sowie an den Menschen, verwendet werden, insbesondere zur oralen Verabreichung von Medikamenten.
Die Applikation von wirkstoffen mittels der erfindungsgemäßen Zubereitungen kann besonders vorteilhaft bei mittelgroßen Haustieren wie Katze, Hund oder Kaninchen, bei kleinen Tieren wie Hamster oder Maus als auch bei großen Tieren wie Großkatzen (Löwe, Tiger) oder auch Nutztieren (Rind, Schaf, Pferd) eingesetzt werden. Die Darreichungsform kann dabei direkt in das Tiermaul appliziert werden, sie kann aber auch mit Hilfe eines Applikators verabreicht werden. Außerdem können die erfindungsgemäßen Zubereitungen unter das Futter gegeben werden, wobei Trockenfutter zu bevorzugen ist (was aber feuchtigkeitshaltige Futter nicht ausschließt).

Geeignete Zusammesetzungen für die Herstellung von erfindungsgemäßen Zubereitungen werden an Hand der nachfolgenden Rezepturen beispielhaft erläutert:

### Beispiel 1:

| | | |
|---|---|---|
| Walocel⁽¹⁾ CRT 30 | 61,2 | Gew.-% |
| Metolose⁽²⁾ 60SH-50 | 8,46 | Gew.-% |
| Wasser⁽⁴⁾ | | |
| Chlorphenamin* | 10 | Gew.-% |
| Propandiol | 5,4 | Gew.-% |
| Mowiol⁽³⁾ 15-79 | 12,24 | Gew.-% |
| Aroma | 2,7 | Gew.-% |

| | | |
|---|---|---|
| *Wirkstoff ⁽¹⁾Natrium-Carboxymethylcollulose ⁽²⁾Handelaname für Hydroxymethylpropylcellulose (HPMC) ⁽³⁾Fa. Hoechat/Aventis AG. ⁽⁴⁾Der Wasser-Anteil (bzw. Wasser-Alkohol-Anteil; Bsp. 3) beträgt bei der Herstellung zwischen 80 und 90 %, gezogen auf den Trocken-Anteil. | | |

### Beispiel 2:

| | | |
|---|---|---|
| Walocel⁽¹⁾ CRT 30 | 82,5 | Gew.-% |
| Chlorphenamin* | 10 | Gew.-% |
| Propandiol | 4,5 | Gew.-% |
| Wasser⁽⁴⁾ | | |
| Aspartam | 3 | Gew.-% |

| | | |
|---|---|---|
| *Wirkstoff ⁽¹⁾Natrium-Carboxymethylcollulose ⁽⁴⁾Der Wasser-Anteil (bzw. Wasser-Alkohol-Anteil; Bsp. 3) beträgt bei der Herstellung zwischen 80 und 90 %, gezogen auf den Trocken-Anteil. | | |

### Beispiel 3:

| | | |
|---|---|---|
| Wasser:Alkohol⁽⁴⁾ | | |
| Wirkstoff | 20 | Gew.-% |
| Polyox 10 | 39,98 | Gew.-% |
| Propandiol-1,2 | 10 | Gew.-% |
| Dexpanthenol | 10 | Gew.-% |
| Menthol | 10 | Gew.-% |
| Eukalyptol | 10 | Gew.-% |
| Azorubin | 0, 02 | Gew.-% |

| | | |
|---|---|---|
| ⁽⁴⁾Der Wasser-Anteil (bzw. Wasser-Alkohol-Anteil; Bsp. 3) beträgt bei der Herstellung zwischen 80 und 90 %, gezogen auf den Trocken-Anteil. | | |

### Beispiel 4:

| | | |
|---|---|---|
| Wasser:⁽⁴⁾ | | |
| Wirkstoff | 15 | Gew.-% |
| Gantrez MS 955 | 25 | Gew.-% |

| | | |
|---|---|---|
| ⁽⁴⁾Der Wasser-Anteil (bzw. Wasser-Alkohol-Anteil; Bsp. 3) beträgt bei der Herstellung zwischen 80 und 90 %, gezogen auf den Trocken-Anteil. | | |

## Patentansprüche

1. Mucoadhäsive Arzneizubereitung zur Verabreichung von Wirkstoffen in der Veterinär- oder Humanmedizin, mit einem Gehalt an mindestens einem Wirkstoff, **dadurch gekennzeichnet, daß** die Zubereitung
- eine mucoadhäsive, in wässrigen Medien zerfallsfähige Matrix aufweist, welche mindestens ein matrixbildendes Polymer enthält und in welcher mindestens ein Wirkstoff gelöst oder dispergiert ist,
- als fester Schaum vorliegt, und
- innerhalb von 15 min nach Applikation auf eine Schleimhautoberfläche zerfällt oder erodiert, sofern sie während dieser Zeit von einem wäßrigen Medium umgeben ist, oder innerhalb von 15 min nach Applikation auf eine Schleimhautoberfläche zunächst geliert und innerhalb von 30 min zerfällt oder erodiert, sofern sie nährend dieser Zeit von einem wäßrigen Medium umgeben ist.

2. Mucoadhäsive Arzneizubereitung nach Anspruch 1, **dadurch gekennzeichnet, daß**, sie flächenförmig ist.

3. Mucoadhäsive Arzneizubereitung nach Anspruch 2, **dadurch gekennzeichnet, daß** ihre Gesamtdicke 5 µm bis 10 mm, bevorzugt 30 µm bis 2 mm und besonders bevorzugt 0,1 mm bis 1 mm beträgt.

4. Mucoadhäsive Arzneizubereitung nach Anspruch 1, **dadurch gekennzeichnet, daß** sie in Form einen Pellets, einer Kapsel oder einer Tablette vorliegt.

5. Mucoadhäsive Arzneizubereitung nach einem der Ansprüche 1 bis 4, **dadurch gekennzeichnet, daß** sie innerhalb von 3 min und besonders bevorzugt innerhalb von 60 s nach Einbringen in ein wässriges Medium zerfällt.

6. Mucoadhäsive Arzneizubereitung nach einem der Ansprüche 1 bis 5, **dadurch gekennzeichnet, daß** sie mindestens einen Hilfsstoff enthält, wobei der/die Hilfsstoffe aus der Füllstoff, Farbstoffe, Sprengmittel, Emulgatoren, Weichmacher, Süßstoffe, Konservierungsmittel, Stabilisatoren, Antioxidantien und Aromastoffe umfassenden Gruppe ausgewählt ist/sind.

7. Mucoadhäsive Arzneizubereitung nach Anspruch 6, **dadurch gekennzeichnet, daß** sie mindestens einen Aromastoff und/oder mindestens einen Süßstoff und/oder mindestens einen Weichmacher enthält.

8. Mucoadhäsive Arzneizubereitung nach einem der Ansprüche 1 bis 7, **dadurch gekennzeichnet, daß** sie mindestens einen Enhancer und/oder mindestens einen durchblutungsfördernden Stoff enthält.

9. Mucoadhäsive Arzneizubereitung nach einem der Ansprüche 1 bis 8, **dadurch gekennzeichnet, daß** sie eine runde oder ellipsenförmige oder ovale Form, oder eine drei-, vier- oder vieleckige Form, oder eine unregelmäßig gerundete Form aufweist.

10. Mucoadhäsive Arzneizubereitung nach einem der Ansprüche 1 bis 9, **dadurch gekennzeichnet, daß** die Dichte des verfestigten Schaumes zwischen 0,01 g/cm³ und 0,8 g/cm³, vorzugsweise zwischen 0,08 g/cm³ und 0,4 g/cm³, und besonders bevorzugt zwischen 0,1 g/cm³ und 0,3 g/cm³ liegt.

11. Mucoadhäsive Arzneizubereitung nach einem der Ansprüche 1 bis 10, **dadurch gekennzeichnet, daß** das/die matrixbildende(n) Polymer(e) aus der Polyvinylalkohol, Cellulosederivate, Stärke und Stärkederivate, Gelatine, Polyvinylpyrrolidon, Gummi arabicum, Pullulan, Acrylate, Polyethylenoxid und Copolymere aus Methylvinyl-Ether und Maleinsäure-Anhydrid umfassenden Gruppe ausgewählt ist/sind, wobei die Gruppe der Cellulosederivate bevorzugt Hydroxypropylmethylcellulose, Hydroxypropylcellulose, Natrium-Carboxymethylcellulose, Methylcellulose, Hydroxyethylcellulose und Hydroxypropylethylcollulose umfaßt.

12. Mucoadhäsive Arzneizubereitung nach einem der Anspruche 1 bis 11, **dadurch gekennzeichnet, daß** es sich bei dem/den Wirkstoff(en) um Wirkstoff(e) handelt, der/die in der veterinärmedizinischen oder humanmedizinischen medikamentösen Therapie verwendet wird/werden, wobei der/die Wirkstoff(e) vorzugsweise aus der Gruppe ausgewählt ist/sind, welche Gastroenterologika, kardiovaskuläre Wirkstoffe, Dermatologika, Gynäkologika, Sexualhormone, Hormone, das hormonale System beeinflussende Wirkstoffe, Antiinfektiva, auf das Muskel- und Skelettsystem wirkende Stoffe, auf das Nervensystem wirkende Stoffe, Analgetika, Anästhetika, Sedativa, Antisedativa, Antiparasitika, auf das Respirationssystem wirkende Stoffe, sowie auf die Sinnesorgane wirkende Stoffe umfaßt.

## Claims

1. Mucoadhesive pharmaceutical preparation for administering active substances in veterinary or human medicine, containing at least one active substance, **characterized in that** the preparation
- is a mucoadhesive matrix disintegratable in aqueous media, which matrix contains at least one matrix-forming polymer and contains at least one active substance which is dissolved or dispersed therein,
- is present in the form of a solid foam, and
- disintegrates or erodes within a period of 15 minutes following application thereof to the surface of a mucous membrane if during this period the preparation is surrounded by an aqueous medium, or initially forms a gel within 15 minutes following application to the surface of a mucous membrane and disintegrates or erodes within a period of 30 minutes if during this period it is surrounded by an aqueous medium.

2. Mucoadhesive pharmaceutical preparation according to claim 1, **characterised in that** it is of a sheetlike nature.

3. Mucoadhesive pharmaceutical preparation according to claim 2 **characterized in that** its total thickness is 5 µm to 10 mm, preferably 30 µm to 2 mm, and more preferably 0.1 mm to 1 mm.

4. Mucoadhesive pharmaceutical preparation according to claim 1, **characterized in that** it is present in the form of pellets, a capsule or a tablet.

5. Mucoadhesive pharmaceutical preparation according to any one of claims 1 to 4, **characterized in that** it disintegrates within 3 min and with particular preference within 60 s after its introduction in an aqueous medium.

6. Mucoadhesive pharmaceutical preparation according to any one of claims 1 to 5, **characterized in that** it contains at least one auxiliary substance, which auxiliary substance(s) is/are selected from the group comprising fillers, colourants, disintegrants, emulsifiers, plasticizers, sweeteners, preservatives, stabilisers, antioxidants and flavours.

7. Mucoadhesive pharmaceutical preparation according to claim 6, **characterized in that** it contains at least one flavour and/or at least one sweetener and/or at least one plasticizer.

8. Mucoadhesive pharmaceutical preparation according to any one of claims 1 to 7, **characterized in that** it contains at least one enhancer and/or at least one substance stimulating the blood flow.

9. Mucoadhesive pharmaceutical preparation according to any one of claims 1 to 8, **characterized in that** it is of round or elliptical or oval shape, or of a triangular, quadrangular or polygonal shape, or of an irregularly rounded shape.

10. Mucoadhesive pharmaceutical preparation according to any one of claims 1 to 9, **characterized in that** the density of the solidified foam is between 0.01 g/cm³ and 0.8 g/cm³, preferably between 0.08 g/cm³ and 0.4 g/cm³, and more preferably between 0.1 g/cm³ and 0.3 g/cm³.

11. Mucoadhesive pharmaceutical preparation according to any one of claims 1 to 10, **characterized in that** the matrix-forming polymer(s) is/are selected from the group comprising polyvinyl alcohol, cellulose derivatives, starch and starch derivatives, gelatine, polyvinyl pyrrolidone, gum arabic, pullulan, acrylates, polyethylene oxide and copolymers of methyl vinyl ether and maleic acid anhydride, the group of the cellulose derivatives preferably comprising hydroxypropylmethyl cellulose, hydroxypropyl cellulose, sodium carboxymethyl cellulose, methyl cellulose, hydroxyethyl cellulose and hydroxypropylethyl cellulose.

12. Mucoadhesive pharmaceutical preparation according to any one of claims 1 to 11, **characterized in that** the active substance(s) is/are active substance(s) which is/are used in drug therapy in veterinary or human medicine, the said active substance(s) preferably being selected from the group comprising gastroenterologic agents, cardiovascular agents, dermatologic agents, gynaecologic agents, sex hormones, hormones, agents having an influence on the hormonal system, anti-infectives, substances acting on the muscular and skeletal systems, substances acting on the nervous system, analgesics, anaesthetics, sedatives, anti-sedative agents, parasiticides, substances acting on the respiratory system, as well as substances acting on the sense organs.

## Revendications

1. Préparation pharmaceutique mucoadhésive pour l'administration de substances actives en médecine vétérinaire ou humaine, possédant une teneur en au moins une substance active, **caractérisée en ce que** la préparation
- présente une matrice mucoadhésive apte à se décomposer dans des milieux aqueux, qui contient au moins un polymère formant une matrice et dans laquelle est dissoute ou dispersée au moins une substance active ;
- est présente sous la forme d'une mousse solide ; et
- subit une décomposition ou une érosion dans un laps de temps de 15 minutes après l'application sur une surface muqueuse, pour autant qu'elle soit entourée par un milieu aqueux pendant ce laps de temps, ou bien se transforme d'abord en gel dans un laps de temps de 15 minutes après l'application sur une surface muqueuse et subit une décomposition ou une érosion dans un laps de temps de 30 minutes, pour autant qu'elle soit entourée par un milieu aqueux pendant ce laps de temps.

2. Préparation pharmaceutique mucoadhésive selon la revendication 1, **caractérisée en ce qu'**elle est de forme plate.

3. Préparation pharmaceutique mucoadhésive selon la revendication 2, **caractérisée en ce que** son épaisseur totale s'élève de 5 µm à 10 mm, de préférence de 30 µm à 2 mm et de manière particulièrement préférée de 0,1 mm à 1 mm.

4. Préparation pharmaceutique mucoadhésive selon la revendication 1, **caractérisée en ce qu'**elle est présente sous la forme d'un pellet, d'une capsule ou d'un comprimé.

5. Préparation pharmaceutique mucoadhésive selon l'une quelconque des revendications 1 à 4, **caractérisée en ce qu'**elle se décompose dans un laps de temps de 3 minutes et de manière particulièrement préférée dans un laps de temps de 60 secondes après son introduction dans un milieu aqueux.

6. Préparation pharmaceutique mucoadhésive selon l'une quelconque des revendications 1 à 5, **caractérisée en ce qu'**elle contient au moins une substance auxiliaire, la/les substances auxiliaires étant choisies parmi le groupe comprenant des substances de remplissage, des colorants, des dispersants, des émulsifiants, des plastifiants, des édulcorants, des conservateurs, des stabilisateurs, des antioxydants et des exhausteurs du goût.

7. Préparation pharmaceutique mucoadhésive selon la revendication 6, **caractérisée en ce qu'**elle contient au moins un exhausteur du goût et/ou au moins un édulcorant et/ou au moins un plastifiant.

8. Préparation pharmaceutique mucoadhésive selon l'une quelconque des revendications 1 à 7, **caractérisée en ce qu'**elle contient au moins un exhausteur et/ou au moins une substance favorisant l'irrigation sanguine.

9. Préparation pharmaceutique mucoadhésive selon l'une quelconque des revendications 1 à 8, **caractérisée en ce qu'**elle présente une forme ronde ou elliptique ou ovale, ou bien une forme triangulaire, carrée ou polygonale, ou encore une forme arrondie de façon irrégulière.

10. Préparation pharmaceutique mucoadhésive selon l'une quelconque des revendications 1 à 9, **caractérisée en ce que** la densité de la mousse solidifiée se situe entre 0,01 g/cm³ et 0,8 g/cm³, de préférence entre 0,08 g/cm³ et 0,4 g/ cm³, et de manière particulièrement préférée entre 0,1 g/cm³ et 0,3 g/cm³.

11. Préparation pharmaceutique mucoadhésive selon l'une quelconque des revendications 1 à 10, **caractérisée en ce que** le/les polymères formant une matrice est/sont choisis parmi le groupe comprenant l'alcool polyvinylique, des dérivés de la cellulose, l'amidon et des dérivés de l'amidon, la gélatine, la polyvinylpyrrolidone, la gomme arabique, le pullulane, des acrylates, l'oxyde de polyéthylène et des copolymères d'éther méthylvinylique et d'anhydride de l'acide maléique, le groupe des dérivés de la cellulose comprenant de préférence l'hydroxypropylméthylcellulose, l'hydroxypropylcellulose, la carboxyméthylcellulose de sodium, la méthylcellulose, l'hydroxyéthylcellulose et l'hydroxypropyléthylcellulose.

12. Préparation pharmaceutique mucoadhésive selon l'une quelconque des revendications 1 à 11, **caractérisée en ce que**, en ce qui concerne la/les substances actives, il s'agit de substances actives que l'on utilise dans la thérapie médicamenteuse de la médecine vétérinaire ou de la médecine humaine, la/les substances actives étant choisies de préférence parmi le groupe qui comprend des médicaments pour la gastro-entérologie, des substances actives cardio-vasculaires, des agents dermatologiques, des agents gynécologiques, des hormones sexuelles, des hormones, des substances actives influençant le système hormonal, des agents anti-infectieux, des substances agissant sur le système musculaire et squelettique, des substances agissant sur le système nerveux, des analgésiques, des anesthésiques, des sédatifs, des antisédatifs, des antiparasitaires, des substances agissant sur le système respiratoire, ainsi que des substances agissant sur les organes sensoriels.
